# EUROPEAN PATENT APPLICATION

(11) **EP 1 862 159 A1**
(43) Date of publication of application: **05.12.2007**
(21) Application number: 06729664.0
(22) Date of filing: 22.03.2006
(51) Int. Cl.: A61K 6/00

(54) **COLORANT COMPOSITION FOR SKIN CARE COSMETIC, FOUNDATION MAKING USE OF THE SAME, AND METHOD OF MAKEUP**

(30) Priority: 23.03.2005 JP 2005083561
(71) Applicant: Shiseido Company, Limited, Chuo-ku Tokyo 104-8010 (JP)
(72) Inventor: KATSUYAMA, Tomoyuki Shiseido Res. Center, Yokohama-shi, Kanagawa 2248558 (JP); TOMOMASA, Satoshi Shiseido Res. Center, Yokohama-shi, Kanagawa 2248558 (JP); HATA, Hideo Shiseido Res. Center, Yokohama-shi, Kanagawa 2248558 (JP)
(74) Representative: Henkel, Feiler & Hänzel
(86) International application number: PCT/JP2006/305697
(87) International publication number: WO 2006/101122

(57) **Abstract**

The object of the invention is to provide a colorant composition for skin cosmetics, that can provide a natural texture while retaining hiding power, a foundation containing the same, and a method of makeup application. A colorant composition for skin cosmetics of the present invention is **characterized in that** the colorant composition, which contains nonwhite coloring materials, does not contain a high refractive index white pigment, and when the spectral reflectance of the colorant composition is measured under the below-described measurement conditions, the minimum spectral reflectance on the white ground in the wavelength range of 630-704 nm is 75% or higher, and the Y value on the white ground is in the range of 25-65. (Measurement conditions) A colorant composition is dispersed in nitrocellulose lacquer, the concentration of the colorant composition is adjusted so that the spectral reflectance on the white ground of a black and white hiding test paper at the wavelength of 400 nm is 10±2%, a black and white hiding test paper is coated with an applicator having a 0.101 mm clearance, and the spectral reflectance is measured with a spectrocolorimeter.

## Description

### RELATED APPLICATIONS

This application claims priority to the Japanese Patent Application 2005-83561 dated on March 23, 2005 and is hereby incorporated with reference for all purposes.

### BACKGROUND OF THE INVENTION

### FIELD OF THE INVENTION

This invention relates to improvement in an colorant composition for skin cosmetics, an foundation and a method of makeup application.

### PRIOR ART

For skin cosmetics such as foundation, a composition that has high hiding power and that is adjusted to a desired skin color is generally used to conceal skin color irregularities, such as pigmented spots and freckles. In order to increase the hiding power, white pigments with a high refractive index such as titanium dioxide and zinc oxide are used. In particular, titanium dioxide has a large light scattering effect and high hiding power because the refractive index is 2.5-2.7 and it is the highest among the powders for cosmetic use.

It has been possible to conceal skin color irregularities, such as pigmented spots and freckles with the use of titanium dioxide. However, when a large amount of titanium dioxide was blended to increase the hiding power, an opaque finish was generated and the natural texture of the bare skin could not be obtained. On the other hand, when the amount of blended titanium dioxide was small, the skin color irregularities such as pigmented spots and freckles could not be sufficiently concealed. Thus, the cosmetics that can provide a natural texture while retaining hiding power have been desired.

The present invention was made in view of the above-described problem, and the object of the invention is to provide a colorant composition for skin cosmetics, that can provide a natural texture while retaining hiding power, a foundation containing the same, and a method of makeup application.

### SUMMARY OF THE INVENTION

The present inventors have initially focused on the light that propagates inside the bare skin. In the light scattering media such as the skin, part of the light irradiated on the skin surface is transmitted to the inside of the skin, and the light is reflected by inner scatterers. As a result, a light is also emitted from different sites other than the irradiated site. The present inventors conducted a study by considering that the distribution of the emission sites is the important factor that decides the texture of the bare skin. As a result, the present inventors found that when a coloring material with a small absorbance in the range of 630-700 nm was blended into skin cosmetics, the distribution of the above-described emission sites approached that of the bare skin, generating a natural texture.

Thus, a colorant composition for skin cosmetics of the present invention is characterized by the colorant composition, which does not contain a high refractive index white pigment, and when the spectral reflectance of the colorant composition is measured under the below-described measurement conditions, the minimum spectral reflectance on the white ground in the wavelength range of 630-700 nm is 75% or higher, and the Y value on the white ground is in the range of 25-65.

### (Measurement conditions)

A colorant composition is dispersed in nitrocellulose lacquer, the concentration of the colorant composition is adjusted so that the spectral reflectance on the white ground of a black and white hiding test paper at the wavelength of 400 nm is 10±2%, a black and white hiding test paper is coated with an applicator having a 0.101 mm clearance, and the spectral reflectance is measured with a spectrocolorimeter.

In the above-described colorant composition, it is preferable that nonwhite coloring materials contained in the colorant composition are plural coloring materials selected from the group consisting of Lithol Rubine BCA, Sunset Yellow FCF, tartrazine, and Hansa Yellow.

Furthermore, a multi-component type foundation of the present invention is characterized in that a white pigment composition containing a high refractive index white pigment and the above-described colorant composition are components.

In the above described multi-component type foundation, it is also preferable that the white pigment composition and the colorant composition are mixed prior to the application to the skin.

Furthermore, a method of makeup application of the present invention is characterized in that the method comprises the application step of a white pigment composition containing a high refractive index white pigment on the skin and the application step of the said colorant composition on the skin.

In the above-described method of makeup application, it is preferable that the amount of an applied high refractive index white pigment on the skin is 0.1-5 mg/cm² in total.

Furthermore, a one-component type foundation of the present invention is characterized in that the foundation contains a nonwhite coloring material and a high refractive index white pigment, and when the spectral reflectance of the foundation-contained portion that is free from the high refractive index white pigment is measured under the below-described measurement conditions, the minimum spectral reflectance on the white ground in the wavelength range of 630-700 nm is 75% or higher, and the Y value on the white ground is in the range of 25-65.

### (Measurement conditions)

The foundation-contained portion that is free from the high refractive index white pigment is dispersed in nitrocellulose lacquer, the concentration of the colorant composition is adjusted so that the spectral reflectance on the white ground of a black and white hiding test paper at the wavelength of 400 nm is 10±2%, a black and white hiding test paper is coated with an applicator having a 0.101 mm clearance, and the spectral reflectance is measured with a spectrocolorimeter.

In the above-described one-component type foundation, it is preferable that the ratio of the total nonwhite coloring material and the high refractive index white pigment that are blended in the foundation (total nonwhite coloring material/(high refractive index white pigment + total nonwhite coloring material)) is 20-90 wt%.

In the above-described one-component type foundation, it is preferable that the amount of blended high refractive index white pigment is 1-25 wt%.

In this specification, a multi-component type foundation indicates the foundation in which a colorant composition and a white pigment composition are separately prepared, and users blend them on the skin or the users mix them before applying to the skin. On the other hand, one-component type foundation indicates the foundation that is prepared into one component as cosmetics.

In the above-described "foundation-contained portion that is free from the high refractive index white pigment" indicates the portion that is prepared without blending a high refractive index white pigment, and the portion other than the high refractive index white pigment is of the same composition as that of the one-component type foundation.

The colorant composition of skin cosmetics of the present invention has high reflectance in the wavelength range of 630-700 nm; as a result, a natural texture can be achieved.

The multi-component type foundation of the present invention contains a white pigment composition containing a high refractive index white pigment and the above-described colorant composition; therefore, it can provide a natural texture while retaining hiding power when both components are applied to the skin.

The method of makeup application of the present invention has the application step of a white pigment composition containing a high refractive index white pigment and the application step of the above-described colorant composition; therefore, it can provide a natural texture while retaining hiding power.

The one-component type foundation of the present invention can provide a natural texture to the made-up skin while retaining hiding power.

### BRIEF DESCRIPTION OF THE DRAWINGS

Fig. 1 is a schematic diagram that shows the spreading emitting sites of light emitted from the inner skin when a light falls on the skin.

Fig. 2 is a graph that shows the intensity distribution of a propagating light when a white light flux was irradiated on the skin.

Fig. 3 is a graph that shows the relationship between the application range of titanium oxide and the intensity distribution of a propagating light.

Fig. 4 shows the respective intensity distributions of propagating light when the absorbance of the cosmetic film is varied.

Fig. 5 is a schematic diagram that explains the relationship between texture of made-up skin and propagating light.

Fig. 6 is the reflection spectra of Sample 3-1 to 3-6 on the white ground.

Fig. 7 is a schematic diagram that explains the reflection spectrum of a colorant composition placed on the white ground.

### DESCRIPTION OF THE PREFERRED EMBODIMENT

In the following section, the preferable mode for carrying out the present invention is described.

The colorant composition for skin cosmetics in the present embodiment is a colorant composition containing nonwhite coloring materials. When the spectral reflectance of the colorant composition is measured under the below-described measurement conditions, the colorant composition is characterized in that the minimum spectral reflectance, on the white ground, in the wavelength range of 630-700 nm is 75% or higher, and the Y value on the white ground is in the range of 25-65. Here, the colorant composition does not contain a high refractive index white pigment.

### (Measurement conditions)

A colorant composition is dispersed in nitrocellulose lacquer, the concentration of the colorant composition is adjusted so that the spectral reflectance on the white ground of a black and white hiding test paper at the wavelength of 400 nm is 10±2%, a black and white hiding test paper is coated with an applicator having a 0.101 mm clearance, and the spectral reflectance is measured with a spectrocolorimeter.

Specific examples of nonwhite coloring materials that satisfy the above-described conditions include aluminum lake, zirconium lake, or barium lake of Amaranth, Erythrosin, New Coccine, Phloxine B, Rose Bengal, Acid Red, Tartrazine, Sunset Yellow FCF, Lithol Rubine B, Lithol Rubine BCA, Lake Red C, Lake Red CBA, Lithol Red, Lithol Red CA, Lithol Red BA, Lithol Red SR, Rhodamine B, Rhodamine B Stearate, tetrachlorotetrabromofluorescein, Toluidine Red, tetrabromofluorescein, Sudan III, Helindone Pink CN, Eosin YS, dibromofluorescein, Permanent Orange, Benzidine Orange G, Orange II, fluorescein, uranine, Quinoline Yellow WS, Quinoline Yellow SS, Benzidine Yellow G, Pyranine Conc, Byrolamine R, Brilliant First Scarlet, Permanent Red F5R, Ponceau SX, Orange I, Orange SS, Hansa Yellow, Naphthol Yellow S, Metanil Yellow, Fast Light Yellow 3G, etc. In addition, it is possible to use these organic colorants, as pigments, by complexing with clay according to the methods in Japanese Unexamined Patent Publication 2004-331877 and Japanese Unexamined Patent Publication 2004-331878. Particularly, in view of cosmetic versatility, it is preferable to use a combination of plural colorants selected from the group consisting of Lithol Rubine BCA, Sunset Yellow FCF, tartrazine, and Hansa Yellow.

The colorant composition for skin cosmetics in the present embodiment is characterized in that the absorbance, at a normally used concentration, is very small in the wavelength range of 630-700 nm.
In this specification, the term "high refractive index white pigment" is defined to be a white pigment with a refractive index of 1.9 or higher. In addition, the white pigment should have a particle size of 0.1-1 µm (particle size of normally used white pigment). Titanium oxide fine powder and zinc oxide fine powder (particle size is of the order of 0.01 µm) used as an ultraviolet shielding agent are excluded. It is preferable to use titanium dioxide as the high refractive index white pigment; however, the particle size should be 0.1-1 µm, which is the normal size for pigment. The particle size of titanium oxide fine powder that is used as an ultraviolet shielding agent is of the order of 0-01 µm. This titanium oxide fine powder has almost no scattering effect in the visible light region, and it will not be included in the white pigment of this specification. The pigments with a refractive index of less than 1.9, such as talc and mica, are usually used as an extender pigment, and satisfactory hiding power cannot be achieved with these pigments as is evident from the below-described test (refer to Table 10). This is because a satisfactory scattering effect cannot be achieved if a difference is small between the refractive index of pigment and that of the material (or air) around the pigment. Thus, it is possible to obtain cosmetics that can provide a natural texture to the made-up skin while retaining hiding power by blending a white pigment with a high refractive index (1.9 or higher).

The multi-component type foundation in the present embodiment is characterized in that the white pigment composition containing a high refractive index white pigment and the above-described colorant composition are both contained in the foundation. A user of a multi-component type foundation applies the white pigment composition and the colorant composition on the skin, separately, and the user adjusts the amount of the colorant composition so that it fits the skin color. The number of colorant compositions is not limited to one (namely, two components: a white pigment composition and a colorant composition), and plural colorant compositions with different color tones may be bundled with a white pigment composition. As described below, however, even a two-component type can satisfactorily meet the needs of various skin colors. In addition to the separate application of the white pigment composition and the colorant composition, the white pigment composition and the colorant composition may be premixed prior to the application to the skin. The color tone of a white pigment composition is not limited to a white color, and a nonwhite coloring material may be blended. For example, the color tone of a white pigment composition can be adjusted to the color tone of a person with light skin color, and the amount of colorant composition applied may be suitably adjusted according to skin color of the user herself or himself. When the nonwhite coloring material is premixed in the white pigment composition, the white pigment composition (excluding the high refractive index white pigment) needs to satisfy similar conditions to those for the above-described colorant composition for skin cosmetics.

The method of makeup application in the present embodiment is characterized in that the method comprises the application step of a white pigment composition containing a high refractive index white pigment on the skin and the application step of the above-described colorant composition on the skin. Here, the order of the application step of the white pigment composition and the application step of the colorant composition is not important.

In the method of makeup application in the present embodiment, the total amount of the applied high refractive index white pigment is preferably 0.1-5 mg/cm². If the amount is less than 0.1 mg/cm², satisfactory hiding power cannot be achieved. If the amount is more than 5 mg/cm², a natural skin feeling cannot be achieved.

The reason for the separate use of a colorant composition and a white pigment composition for the foundation is as follows. The coloring material used in the present invention does not have absorption in the range of 630-700 nm, and the color tone is different from those of thus far used foundations, which were prepared using iron oxide coloring materials. Generally, in addition to red, yellow, and white coloring materials, a foundation contains a black coloring material, which has absorption in the range of 630-700 nm, to adjust the chroma. Thus, it is possible to preadjust the color close to that of the bare skin color. Accordingly, even when the foundation is directly applied to the skin, there is hardly an uncomfortable feeling due to the color difference. However, the colorant composition of the present invention does not have absorption in the range of 630-700 nm, and the color tone cannot be matched to that of the skin color. As a result, the direct application may give a very uncomfortable feeling. In such a case, it is possible to eliminate an uncomfortable feeling due to the color difference by separating the application step of a colorant composition and the application step of a white pigment composition. The adoption of such a method as a makeup technique is desirable.

However, there are occasions in that a single application is more desirable for users. In such occasions, the above-described colorant composition (high refractive index white pigment is not contained) and a white pigment composition may be mixed, prior to the application of the foundation, so that the foundation matches each person's skin.

A multi-component type foundation, in which a colorant composition without a high refractive index white pigment and a white pigment composition containing a white pigment are separate components, was described above. The technology of the present invention is also applicable to a foundation that is manufactured into one unit (one-component type foundation) as is the case for a conventional foundation. A one-component type foundation in the embodiment of the present invention is characterized as follows. The foundation contains a nonwhite coloring material and a high refractive index white pigment. When the spectral reflectance is measured under the below-described measurement conditions for the portion in which the high refractive index white pigment in the above-described one-component type foundation is excluded, the minimum spectral reflectance, on the white ground, in the wavelength range of 630-700 nm is 75% or higher, and the Y value on the white ground is in the range of 25-65.

### (Measurement conditions)

The foundation-contained portion that is free from the high refractive index white pigment is dispersed in nitrocellulose lacquer, the concentration of the colorant composition is adjusted so that the spectral reflectance on the white ground of a black and white hiding test paper at the wavelength of 400 nm is 10±2%, a black and white hiding test paper is coated with an applicator having a 0.101 mm clearance, and the spectral reflectance is measured with a spectrocolorimeter.
The ratio (total nonwhite coloring material/(total high refractive index white pigment + total nonwhite coloring material)), which indicates the content of the nonwhite coloring material with respect to the total coloring material blended in the one-component type foundation, is preferably 20%-90%, and more preferably 20-70%. If the blending ratio is less than 20%, the applied color becomes whitish and the makeup color becomes unnatural. On the other hand, if the blending ratio is more than 90%, the applied color becomes reddish. Thus, it is necessary to thinly apply the foundation; as a result, satisfactory hiding power cannot be achieved. If the blending ratio is 20%-70%, a more natural makeup color can be achieved.

The amount of the white pigment of high refractive index blended in the one-component type foundation is preferably 1-25 wt%. When the content is less than 1%, the hiding power is not satisfactory. When the content is higher than 25%, the hiding power is satisfactory; however, the makeup color is somewhat whitish for a person with a deep skin color.

The form of foundation is not limited in particular, and applicable forms can be found in solid form, powdery form, oily stick form, W/O or O/W emulsion form, etc.

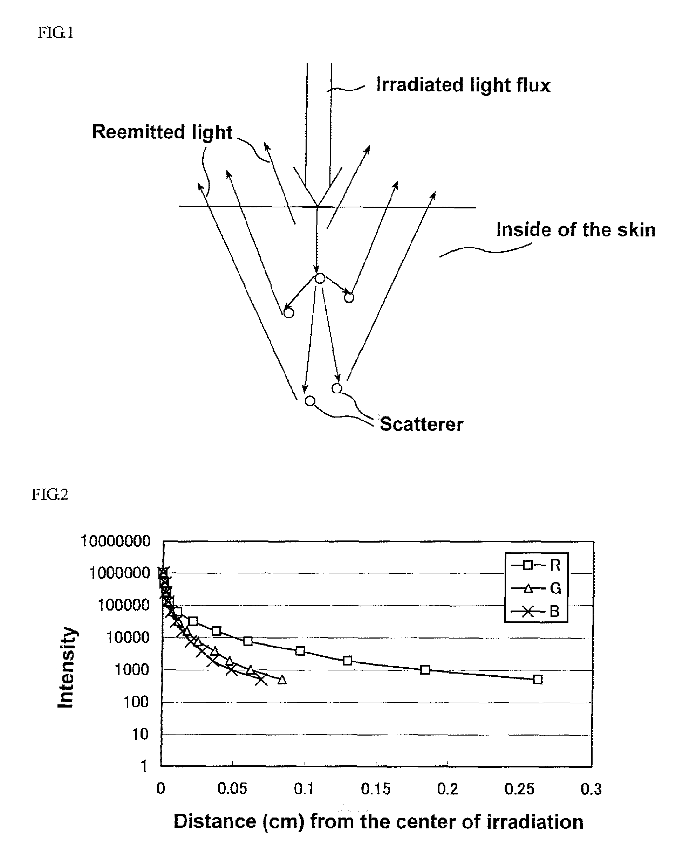

An outline for the constitution of the present invention has been described above, and the detailed description is given below. In the process for reaching the present invention, the present inventors focused on the intensity distribution of a propagating light on the bare skin. Thus, the present inventors focused on the distribution of emitting light on the reflection surface around the irradiated point. In the scattering media such as the skin, incident light is emitted from different sites from the incident site. As shown schematically in Fig. 1, an irradiated light flux is transmitted into the skin and reflected or scattered by scatterers in the skin. Because the reflection sites and scattering sites inside the skin are distributed along the depth direction, a reemitted light from the inside of the skin comes out from the different sites than the initial irradiated site. The present inventors considered that the distribution of the emitting sites from the inside of the skin was the major factor in determining texture of the bare skin. Thus, the present invention was achieved by finding the conditions that generate the distribution close to that of the bare skin when skin cosmetics were applied.

Fig. 2 is a graph that shows the intensity distribution of a propagating light when a white light flux with a radius of 0.5 mm was irradiated on the skin. Here, the abscissa is the distance (cm) from the center of irradiation, and the ordinate is the intensity of reflected light. As seen from the graph in Fig. 2, the contribution of a red light (wavelength range: 630-700 nm) to the intensity of a reflected light (intensity of a propagating light), excluding the light from the center of irradiation, is very large. Thus, the intensity distribution of a propagating light in the wavelength range of 630-700 nm can be inferred to have a large effect on the texture of the skin. Therefore, the present inventors conducted a preliminary experiment to investigate the conditions for skin cosmetics that generate an intensity distribution of a propagating light close to that of the bare skin. The experiment was conducted by focusing on a red light, in particular, that is a light in the wavelength region of 633 nm.

Initially, a 5% Vaseline P dispersion of pigment grade titanium dioxide (Tronox RKB2, Bayer) was tested. The applied amount (mg/cm²) was varied as shown in the below-described Table l, and the intensity distribution of a propagating light was measured. The total transmittance (direct transmittance + diffuse transmittance) for each applied amount is also shown in Table 1. The experimental conditions were as follows. The medial side of the forearm part (5×5 cm²) was used as the application area, a HeNe laser (wavelength: 633 nm) was used as a light source, and an irradiated light flux radius of 0.5 mm was used.

**(Table 1)**

| | | | | | | |
|---|---|---|---|---|---|---|
| Applied amount (mg/cm²) | 0 (bare skin) | 1 | 2 | 3 | 5 | 10 |
| Total transmittance | - | 80% | 60% | 55% | 30% | 8% |

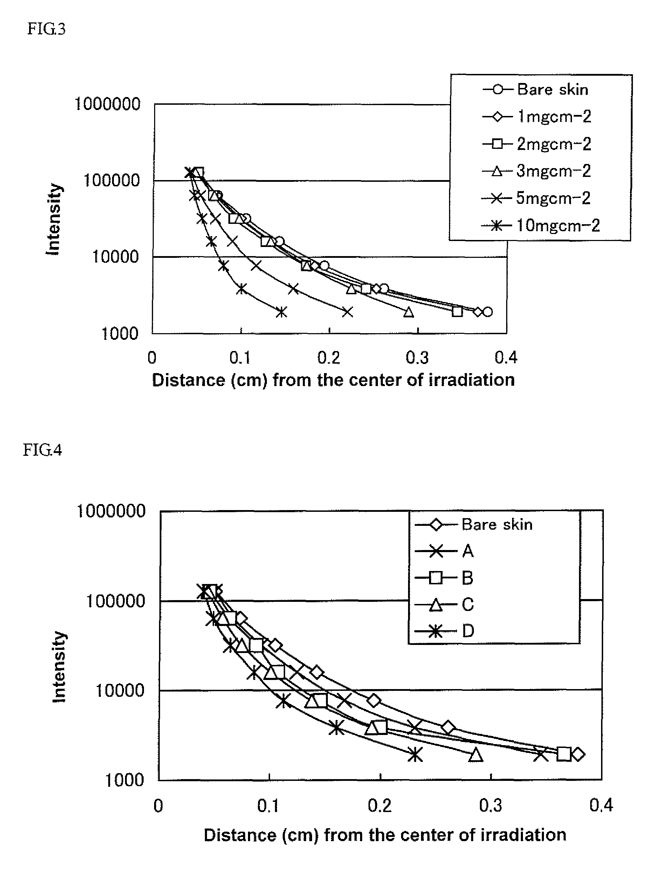

The results of the above experiment are shown in Fig. 3. Here, the abscissa is the distance (cm) from the center of irradiation, and the ordinate is the intensity of a reflected light. As seen from the graph in Fig. 3, when the applied amount is 5 mg/cm² or more, the propagating light intensity at the points away from the irradiated point decreased significantly. However, in the normal application range (1 mg/cm² -5 mg/cm²) of foundation, there is hardly a change, in the propagating light intensity, from that of the bare skin. Thus, when the total transmittance is at least 55% or higher, as seen from Table 1, the light scattering effect of titanium dioxide does not affect the propagating light intensity distribution very much.

In the following section, the effect of light absorption of coloring material to the propagating light was investigated. As shown in Table 2, zero wt%, 0.125 wt%, 0.25 wt%, or 0.5 wt% of black iron oxide was added to the above-described 5% Vaseline P dispersion of pigment grade titanium dioxide (Compositions A to D). The applied amount was adjusted so that each total transmittance is nearly constant (60%), and the intensity distribution of a propagating light was investigated.

**(Table 2)**

| Composition | A | B | C | D |
|---|---|---|---|---|
| Applied amount (mg/cm²) | 3 | 3 | 2 | 2 |
| Total transmittance | 60% | 60% | 60% | 60% |

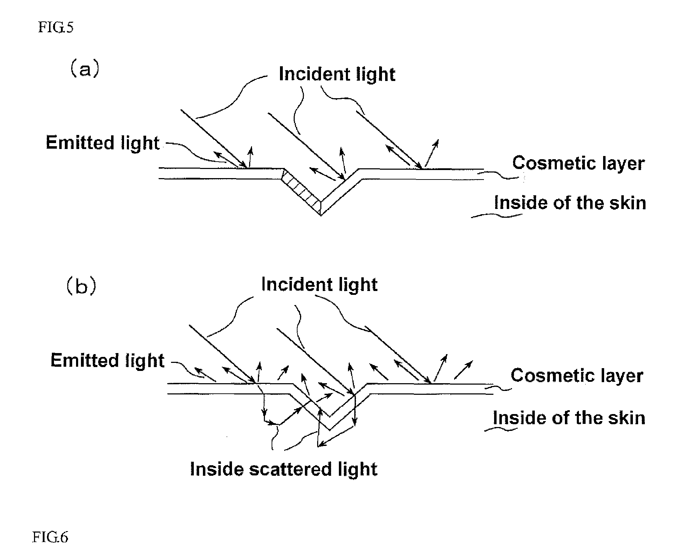

The results are shown in Fig. 4, the abscissa is the distance (cm) from the center of irradiation, and the ordinate is the intensity of reflected light. From the results shown in Fig. 4, we can see that the intensity of propagating light significantly decreases by blending black iron oxide. This indicates that even when the transmittance of the applied layer is about the same, the propagating light intensity is strongly affected because of the light absorption by the applied layer.

Thus, it was found that the effect of light absorption by a coloring material to the intensity distribution of the propagating light is large under the conditions normally used for skin cosmetics such as foundation. This may be explained in the following way. As shown in Fig. 1, an incident light is scattered and absorbed in the skin, and reemitted to the outside of the skin. When cosmetics are applied to the skin, the amount of incident light that goes into the skin decreases because the light scattering and light absorption by the applied film take place. An incident light flux that enters into the skin is scattered and spread inside the skin. A part of the reemitted light from the skin is transmitted through the applied film, and another part is reflected again from the applied film and reenters into the skin. The light reflected again is scattered inside the skin, a part of that will be transmitted through the applied film, and another part is reflected again; this process is repeated. In the case of an applied film with large light scattering and small light absorption (corresponding to the case in which only the above-described titanium dioxide is blended), almost no absorption takes place when a light transmit through the applied film (at the entering time into the skin and at the exiting time from the skin). In addition, a decrease in light intensity due to absorption hardly takes place when the light is reflected again on the applied film and reenters the skin. As a result, a decrease in the propagating light intensity is suppressed. In the case of an applied film in which the extent of light scattering is the same to the above case but it has large light absorption (corresponding to the case in which black iron oxide is added to titanium dioxide), the following events take place. In addition to the attenuation of light due to the light absorption at the entering time into the skin and at the exiting time from the skin, the light intensity attenuates due to the absorption by the applied film during the second reflection. Therefore, the further apart from the incident site, the larger the attenuation of the propagating light intensity.

### Concerning optical properties of coloring materials used in colorant compositions and the quality of makeup finish

Based on the above-described results, samples were prepared by using Lithol Rubine BCA (Toyo Ink Mfg. Co.) as a red coloring material and tartrazine aluminum lake (Sun Chemical Corporation) as a yellow coloring material. They were blended in the colorant composition to conduct tests. For comparison, samples were prepared using red iron oxide, yellow iron oxide, and black iron oxide, which are generally used in the foundation. The results are shown in Table 3 for the composition (wt%), color tone of the colorant composition (before mixing with titanium dioxide), color tone of made-up skin after the application of cosmetics, hiding power, and the sensory evaluation of natural texture.

**(Table 3)**

| | | 3-1 | 3-2 | 3-3 | 3-4 | 3-5 | 3-6 |
|---|---|---|---|---|---|---|---|
| Colorant composition | Liquid paraffin | 84% | 83% | 77.5% | 83.5% | 82.5% | 78.5% |
| | Lithol Rubine BCA | - | - | 0.25% | 0.25% | - | 0.25% |
| | Tartrazine-aluminum lake | - | - | 9.75% | - | 9.75% | 9.75% |
| | Red iron oxide | 2% | 2% | - | - | 1% | - |
| | Yellow iron oxide | 4% | 4% | - | 4% | - | - |
| | Black iron oxide | - | 1% | 1% | - | - | - |
| Color tone of a composition before mixing with a Brown white pigment | | Brown | Brown | Brown | Dark red | Dark red | Bright red |
| White pigment | Titanium dioxide | 10% | 10% | 10% | 10% | 10% | 10% |
| Color tone (before the application to the skin) of a composition after mixing with a white pigment | | ○ | ⊚ | ⊚ | Δ | Δ | × |
| Hiding power | | ⊚ | ⊚ | ⊚ | ⊚ | ⊚ | ⊚ |
| Natural texture | | × | × | × | × | × | ⊚ |

(Evaluation criteria for the color tone (before the application to the skin) of a composition after mixing with a white pigment)
⊚: very close to the skin color
○: somewhat close to the skin color
Δ: somewhat different from the skin color
×: different from the skin color
(Evaluation criteria for the hiding power)
⊚: concealed
○: somewhat concealed
Δ: barely concealed
×: not concealed
(Evaluation criteria of natural texture)
⊚: even and natural finish with a transparent feeling
○: somewhat natural finish
Δ: somewhat unnatural finish
×: unnatural finish

Sample 3-2 is skin cosmetics in which red iron oxide, yellow iron oxide, and black iron oxide, which are general coloring materials, were used. Sample 3-6 is a colorant composition that corresponds to the embodiment of the present invention, and Lithol Rubine BCA and tartrazine, which are coloring materials with little absorption in the range of red light, were used. Sample 3-3 was prepared by blending black iron oxide to Sample 3-6, and the color tone of the colorant composition itself (after mixing with a white pigment) was adjusted to that of the skin. Sample 3-1 was Sample 3-2 colorant composition minus black iron oxide. Sample 3-4 was obtained by blending yellow iron oxide instead of tartrazine in Sample 3-6. Sample 3-5 was obtained by blending red iron oxide instead of Lithol Rubine BCA in sample 3-6.

The sensory evaluation tests were conducted by mixing the same amount of titanium dioxide to the colorant composition of each sample in Table 3 and by applying the mixture to the skin. As seen from Table 3, there was no difference in the hiding power itself because the amounts of applied titanium dioxide were the same. However, in the evaluation of natural texture of made-up skin, Sample 3-6 exhibited an excellent effect, but all others exhibited a poor effect. Sample 3-6 does not contain black iron oxide, and the color tone of the colorant composition itself was a bright red color. Even after the addition of titanium dioxide, the color tone was very different from that of the skin color. As seen from the below-described results in Table 4, however, the color could be made natural when it was applied to the skin.

When a comparative observation was made between the made-up skin with Sample 3-1 (traditional cosmetics) and the made-up skin with Sample 3-6, there was a difference in the appearance of the skin contour and shadow. With the samples with a low propagating light intensity (Samples 3-1 to 3-5), the skin surface was uneven, and the shadow section on the skin was black and deep-colored. On the other and, with the sample with a high propagating light intensity (Sample 3-6), the skin surface was even, and the shadow was light red. As shown in Fig. 5(a), with the traditional cosmetics, the intensity of the emitted light from the sites away from the irradiated site is low; therefore, a shadow is considered to clearly appear at the shaded area of Fig. 5(a). However, because cosmetics 3-6 have a propagating light intensity that is close to the bare skin, as shown in Fig. 5(b), an incident light at other sites is scattered inside the skin, and a light is emitted from the site without incident light (shadow section). As a result, a shadow due to the skin contour is close to that of the bare skin, and a natural texture is generated.

Compositions (colorant composition before mixing with a white pigment) of Samples 3-1 to 3-6 were respectively coated on a black and white hiding test paper, and the obtained reflection spectra on the white ground are shown in Fig. 6. The reflection spectra were measured under the following conditions.

### (Measurement conditions)

1.0g of a colorant composition was dispersed in 15g of nitrocellulose lacquer (Musashi Paint Co., product name: Nitronclear), and the dispersed system was coated on a black and white hiding test paper with an applicator having a 0.101 mm clearance. The reflection spectrum was measured with a spectrocolorimeter (Konica Minolta, product name: CM2600d) under the conditions of an aperture diameter of 11 mmØand the SCE (specular component excluded) mode. As the black and white hiding test paper, a Leneta Form 5C opacity chart with the size of 7-5/8 × 10-1/4 in (194 x 260 mm) was used.

As seen from Fig. 6, for Sample 3-6 with which the made-up skin showed a natural texture, the minimum spectral reflectance on the white ground was 75% or higher in the wavelength range of 630-700 nm (spectral reflectance at the wavelength of 400 nm was about 10%). Judging from these, the absorption in the wavelength range of 630-700 nm is small. On the other hand, the light absorption in the above-described wavelength region is large (reflectance is small) for other samples with which the made-up skin showed an unnatural texture. Thus, the higher the reflectance in the above-described wavelength region, the higher the effect of generating a natural texture for made-up skin. This also proves that the propagating light intensity in the red light region (wavelength 630-700 nm) affects the natural texture of made-up skin. Thus, it was found that a natural texture could be obtained with composition 3-6.

In a general foundation, a black coloring material that has absorption in the entire wavelength region of visible light is almost always added to adjust chroma and lightness. Thus, the color is adjusted to roughly the same color as that of the bare skin color, and a difference is hardly felt between the applied color and the bare skin color. However, the composition in Sample 3-6 has little light absorption in the wavelength range of 630-700 nm. Therefore, the color tone of the composition after mixing with a high refractive index white pigment also has extremely high lightness and chroma compared with the color tone of the general foundation. As a result, depending upon subjects, it has sometimes been difficult to adjust the applied color to a natural color.

In these circumstances, the present inventors have diligently studied the application method of a composition with high chroma and high lightness, such as Sample 3-6, without generating an uncomfortable feeling. As a result, the present inventors found that the applied color can be relatively easily allowed to be natural for various skin colors by separately applying a colorant composition without a high refractive index white pigment and a white pigment composition with a high refractive index white pigment. A colorant composition of Sample 3-6 in Table 3 after mixing with a white pigment was designated to be 4-1, and a colorant composition before mixing with a white pigment was designated to be 4-2. Composition 4-1 was directly applied to four subjects, and Composition 4-2 was applied to the four subjects after the white pigment composition was applied. Then the applied colors were compared and the obtained results are shown in Table 4.

**(Table 4)**

| Subject | | 4-1 | | White pigment composition + 4-2 | | |
|---|---|---|---|---|---|---|
| | Skin color tone | Applied amount of 4-1 | Applied color | Applied amount of white pigment composition | Applied amount of 4-2 | Applied color |
| A | Whitish | 0.5 mg/cm² | ⊚ | 0.5 mg/cm² | 0.4 mg/cm² | ⊚ |
| B | Reddish | 0.5 mg/cm² | Δ | 0.5 mg/cm² | 0.8 mg/cm² | ⊚ |
| C | Yellowish | 0.5 mg/cm² | Δ | 0.5mg/cm² | 0.6 mg/cm² | ⊚ |
| D | Blackish | 0.5 mg/cm² | × | 0.5 mg/cm² | 1.0 mg/cm² | ⊚ |

### (Preparation method of a white pigment composition)

Titanium dioxide was dispersed in Vaseline, and the dispersion was kneaded three times with a three-roller mill to obtain a sufficiently dispersed white pigment composition. The white pigment composition was 10 wt% titanium dioxide (Tronox RKB2, Bayer) and 90 wt% Vaseline.

### (Test method)

Composition 4-1 (0.5 mg/cm²) was applied to four subjects. Separately, after the application of the white pigment composition, a suitable amount of Composition 4-2 was applied to the four subjects so that the applied color would be natural. The applied colors were judged by visual examination.
(Evaluation criteria for the applied color)
⊚: natural
○: acceptably natural
Δ: somewhat unnatural
×: obviously unnatural

As shown in Table 4, it was found that a natural color can be achieved by adjusting the amount of the applied colorant composition depending upon the subjects. The results may be explained in the following way. The human skin color varies from person to person, and a color close to the skin color is achieved by a suitable mixing of the scattering of white color by keratin and collagen in the stratum corneum and dermis and the red component (includes yellow) due to hemoglobin and bilirubin in the blood. In addition, chroma and lightness are lowered by a brown to black component of melanin. Accordingly, the essential determining factors of the skin color tone are a red component due to the blood and a white component due to scattering. If the ratio is suitably selected to each user, the natural color would be formed even when the chroma and lightness is somewhat different from those of the bare skin. Actually, Sample 3-6, which is constituted with a coloring material that has no absorption in the range of 630-700 nm, has a bright red color close to the blood before mixing with a high-refractive pigment. When iron oxide coloring materials, which are generally used in foundations, are used as shown for 3-1 to 3-5, a bright red color cannot be achieved. In the composition of the present invention, a coloring material constitution that imitates the natural skin color constitution is adopted; as a result, the color intimate to skin color is considered to be achieved without using a black color.

In addition, it has become possible to adjust the color to various skin colors of many users because the application can be performed while adjusting the amount of the colorant composition by separating a colorant composition (red component) without a high refractive index white pigment and a composition with a high refractive index white pigment (white component). This type of concept has been non-existent in the past, and it has been the premise to match the composition of a foundation to the bare skin color. Thus, it has been considered that a black pigment is an essential component of foundation. In the red colorant composition, a red coloring material and a yellow coloring material are contained. As described below, the skin color tone varies depending upon the above combination when a white pigment is mixed. Accordingly, there are numerous combinations when a red coloring material, a yellow coloring material, and a white pigment are varied. However, the number of combinations can be markedly decreased by separation into a colorant composition and a composition containing a white pigment, and it is sensible to separate a foundation into two components.

In the following section, the optical properties, which should be satisfied by a colorant composition without a high refractive index white pigment, are explained.

### Optical properties of a colorant composition

Here, optical properties of a suitable coloring material, which is used in a colorant composition of a foundation of the present embodiment, are discussed. The conditions necessary for the colorant composition of the present invention are: (1) no absorption in the range of 630-700 nm, and (2) this colorant composition changes to skin color when mixed with a white pigment, as described above.

The most suitable general method to observe these properties is to coat a black and white hiding test paper using a suitable concentration and estimate the properties from the obtained spectral reflectance curve. Because the types and concentrations of coloring materials in the colorant composition are all different, it is necessary to standardize the amount of coating with a suitable method. The skin color belongs to the hue YR; therefore, the colorant composition consists of a reddish to yellowish coloring material or a mixture thereof. This indicates that the colorant composition absorbs blue to green light and that the spectral reflectance curve of a coat on the white ground is basically an increasing curve. In order to standardize the amount of coating, on a hiding test paper, with a colorant composition, a coating is applied so that the reflectance at 400 nm is 10±2%. If the post-coating spectral reflectance on the white ground has increased or barely decreased, in the range of 630-700 nm, from the pre-coating spectral reflectance on the white ground, the absorption by the colorant composition at the corresponding wavelengths is determined to be negligible.

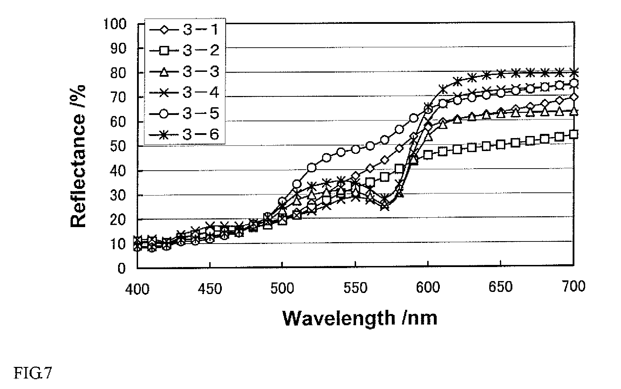

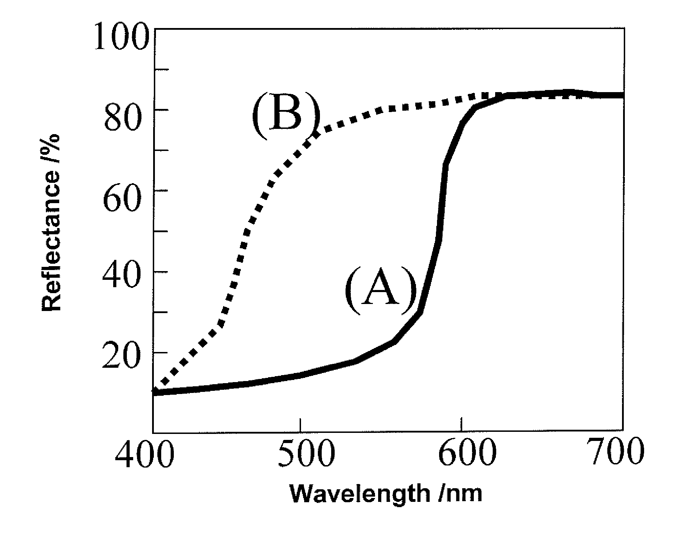

In the following section, the necessary conditions under which a skin color is generated, when a colorant composition is mixed with a white coloring material, are discussed. According to the present coating method, the reflectance at 400 nm and the reflectance at 700 nm on the white ground are both fixed. If the reflectance distribution in the range of 400-700 nm is low on the whole as shown in graph (A) of Fig. 7, the colorant composition is very reddish, and it is expected that a red shift takes place when mixed with a white pigment. On the other hand, if the reflectance is high on the whole as shown in graph (B) of Fig. 7, the colorant composition is yellowish, and it is expected that it becomes yellowish when mixed with a white pigment. Thus, it is reasoned that the reflectance distribution with a suitable magnitude is necessary. The magnitude of the reflectance distribution can be replaced with the Y value of the three stimulus values (2° visual field, D65, JIS Z-8701, Z-8722) as an indicator.

A suitable range of the above-described Y value is discussed. As shown in Tables 5 to 8, colorant compositions with various blending percentages (wt%) were prepared, and a coating was applied so that the reflectance at 400 nm was 10±2%. Then the minimum value of the spectral reflectance in the range of 630-700 nm and the Y value were measured. In addition, the naturalness as a skin color was evaluated when a white pigment composition was applied to the skin and then each colorant composition was applied over that.

(Evaluation criteria for the naturalness as skin color)
⊚: natural as skin color
○: reasonably natural as skin color
ΔY: somewhat yellowish as skin color
ΔR: somewhat reddish as skin color
×Y: too yellow as skin color
×R: too red as skin color

### (Colorant compositions)

### 1) Lithol Rubine BCA-tartrazine system, Part 1

Lithol Rubine BCA (Toyo Ink Mfg. Co.) and tartrazine aluminum lake (Sun Chemical Corporation) were used as coloring materials, and samples were prepared according to the compositions shown in Table 5.

**(Table 5)**

| | 5-1 | 5-2 | 5-3 | 5-4 | 5-5 |
|---|---|---|---|---|---|
| Lithol Rubine BCA | 1.7 | 0.7 | 0.5 | 0.3 | 0.2 |
| Tartrazine aluminum lake | 18.3 | 19. 3 | 19.5 | 19.7 | 19.8 |
| Glyceryl tri(2-ethylhexanoate) | 50 | 50 | 50 | 50 | 50 |
| Vaseline | 30 | 30 | 30 | 30 | 30 |
| Applied color | ΔR | ○ | ⊚ | ○ | ΔY |
| Spectral reflectance at 400 nm (on the white ground) | 10 | 10 | 10 | 10 | 10 |
| Y value (on the white ground) | 26 | 38 | 50 | 55 | 60 |
| Minimum value of spectral reflectance in the range of 630-700nm (on the white ground) | 80 | 80 | 80 | 80 | 80 |

### 2) Lithol Rubine BCA-tartrazine system, Part 2

Lithol Rubine BCA (Toyo Ink Mfg. Co.) and tartrazine aluminum lake (Kishi Kasei Co.) were used as coloring materials, and samples were prepared according to the compositions shown in Table 6.

**(Table 6)**

| | 6-1 | 6-2 | 6-3 | 6-4 | 6-5 | 6-6 | 6-7 |
|---|---|---|---|---|---|---|---|
| Lithol Rubine BCA | 0.5 | 0.4 | 0.33 | 0.23 | 0.17 | 0.10 | 0.08 |
| Tartrazine aluminum lake | 9.5 | 9.6 | 9.67 | 9.77 | 9.83 | 9.90 | 9.92 |
| Glyceryl tri(2-ethylhexanoate) | 60 | 60 | 60 | 60 | 60 | 60 | 60 |
| Vaseline | 30 | 30 | 30 | 30 | 30 | 30 | 30 |
| Applied color | ΔR | ΔR | ΔR | ○ | ⊚ | ○ | ○ |
| Spectral reflectance at 400 nm (on the white ground) | 10 | 10 | 10 | 9 | 10 | 10 | 9 |
| Y value (on the white ground) | 27 | 29 | 30 | 33 | 36 | 44 | 45 |
| Minimum value of spectral reflectance in the range of 630-700nm (on the white ground) | 80 | 79 | 81 | 79 | 79 | 79 | 79 |

### 3) Sunset Yellow FCF-tartrazine system

Sunset Yellow FCF (Sun Chemical Corporation) and tartrazine aluminum lake (Sun Chemical Corporation) were used as coloring materials, and samples were prepared according to the compositions shown in Table 7.

**(Table 7)**

| | 7-1 | 7-2 | 7-3 | 7-4 | 7-5 | 7-6 | 7-7 |
|---|---|---|---|---|---|---|---|
| Sunset Yellow FCF | 1.13 | 3.29 | 432 | 5.33 | 6.32 | 7.27 | 8.21 |
| Tartrazine aluminum lake | 8.87 | 6.71 | 5.68 | 4.67 | 3.68 | 2.73 | 1.79 |
| Glyceryl tri(2-ethylhexanoate) | 50 | 50 | 50 | 50 | 50 | 50 | 50 |
| Vaseline | 40 | 40 | 40 | 40 | 40 | 40 | 40 |
| Applied color | ΔY | ○ | ○ | ⊚ | ⊚ | ○ | ○ |
| Spectral reflectance at 400 nm (on the white ground) | 10 | 10 | 10 | 10 | 10 | 9 | 9 |
| Y value (on the white ground) | 60 | 51 | 48 | 46 | 45 | 44 | 42 |
| Minimum value of spectral reflectance in the range of 630-700nm (on the white Bound) | 80 | 80 | 80 | 80 | 80 | 80 | 80 |

### 4) Sunset Yellow FCF- Hansa Yellow system

Sunset Yellow FCF (Sun Chemical Corporation) and Hansa Yellow (Kishi Kasei Co.) were used as coloring materials, and samples were prepared according to the compositions shown in Table 8.

**(Table 8)**

| | 8-1 | 8-2 | 8-3 | 8-4 | 8-5 | 8-6 | 8-7 |
|---|---|---|---|---|---|---|---|
| Sunset Yellow FCF | 2 | 2.6 | 3.1 | 3.5 | 3.9 | 4.3 | 4.7 |
| Hansa Yellow | 3 | 2.4 | 1.9 | 1.5 | 1.1 | 0.7 | 0.3 |
| Glyceryl tri(2-ethylhexanoate) | 45 | 45 | 45 | 45 | 45 | 45 | 45 |
| Vaseline | 50 | 50 | 50 | 50 | 50 | 50 | 50 |
| Applied color | ΔY | ○ | ○ | ⊚ | ⊚ | ○ | ○ |
| Spectral reflectance at 400 (on the white ground) | 10 | 10 | 10 | 10 | 10 | 9 | 9 |
| Y value (on the white ground) | 60 | 51 | 48 | 46 | 45 | 44 | 42 |
| Minimum value of spectral reflectance in the range of 630-700nm. (on the white ground) | 80 | 80 | 80 | 80 | 80 | 80 | 80 |

### Preparation method of a white pigment composition

Titanium dioxide was dispersed in Vaseline, and the dispersion was kneaded three times with a three-roller mill to obtain a sufficiently dispersed white pigment composition. The white pigment composition was 10 wt% titanium dioxide (Tronox RKB2, Bayer) and 90 wt% Vaseline.

### Preparation method of colorant composition

Respective coloring materials (30 wt%) were dispersed in glyceryl tri(2-ethylhexanoate), kneaded three times with a three-roller mill to sufficiently disperse, and the dispersion was dispersed in the rest of glyceryl tri(2-ethylhexanoate). Then Vaseline was added to the dispersion, dissolved by heating, cooled in a water bath with stirring, and the obtained gel-like material was used as a sample.

### Identification method of applied color

The white pigment composition was applied to the skin (1 mg/cm²), each colorant composition was applied over that, and the color tone of the applied color was evaluated by cosmetic technologists.

### Measurement methods for the spectral reflectance at 400 nm and 630 nm-700 nm and for the Y value

To each colorant composition (1 g in for formulation 1), 1.6 g for formulation 2), 1 g for formulation 3), and 1 g for formulation 4)) was added nitrocellulose lacquer (Musashi Paint Co., product name: Nitronclear), respectively, the total weight was made 16 g, and the mixture was sufficiently kneaded. Then the material was applied with a 0.101 mm applicator on a black and white hiding test paper (Leneta Form 5C opacity chart, size: 7-5/8×10-1/4 in (194 x 260 mm)). The spectral reflectance at 400 nm and the (average) spectral reflectance at 630 nm and higher was determined with a spectrocolorimeter (Minolta Co., product name: CM2600d) under the SCE (specular component excluded) mode and the aperture diameter of I1 mm, and the Y value was also determined. That is, the thickness of the applied film was kept constant, and the concentration of the colorant composition in the applied film was adjusted so that the spectral reflectance at 400 nm was 10±2%.

From the results in Tables 5 to 8, it was found that the larger the Y value, the more yellowish the color, and the smaller the Y value, the more reddish the color though the suitable range varies depending upon coloring materials. In addition, although the suitable range of the Y value varies depending upon the combination of coloring materials, it was found that the color changed to the skin color, when mixed with a white pigment, if the range was adjusted to roughly 25-65. If the range was 30-60, a more natural skin color could be achieved when applied to the skin after being mixed with a white pigment.

### Amount of an applied high refractive index white pigment

In the following section, the desirable amount of a high refractive index white pigment used in the makeup application of the present invention was investigated.

A white pigment composition was prepared by dispersing Pigment grade titanium dioxide (Tronox RKB2, Bayer) in Vaseline and by kneading three times with a three-roller mill to achieve sufficient dispersion (the white pigment composition was 10 wt% titanium dioxide and 90 wt% Vaseline). As the colorant composition, Sample 3-6 (before mixing with a white pigment) in Table 3 was used.

The amount of the applied white pigment composition was varied, and the colorant composition was applied until immediately before the post-application color tone becomes unnatural. The sensory evaluation results for the hiding power and natural texture are shown.

**(Table 9)**

| | | | | | | | | | |
|---|---|---|---|---|---|---|---|---|---|
| Applied amount of titanium oxide (mg/cm²) | 0.01 | 0.05 | 0.1 | 1.0 | 2.0 | 3.0 | 4.0 | 4.5 | 5.0 |
| Hiding power | × | × | ○ | ⊚ | ⊚ | ⊚ | ⊚ | ⊚ | ⊚ |
| Natural texture | ⊚ | ⊚ | ⊚ | ⊚ | ⊚ | ⊚ | ⊚ | ○ | × |

(Evaluation criteria for the hiding power)
⊚: concealed
○: somewhat concealed
Δ: barely concealed
×: not concealed
(Evaluation criteria of natural texture)
⊚: even and natural finish with a transparent feeling
○: somewhat natural finish
Δ: somewhat unnatural finish
×: unnatural finish

As seen from Table 9, if the amount of applied titanium dioxide was 0.1 mg/cm² or less, satisfactory hiding power could not be achieved. If it was 5 mg/cm² or more, the naturalness of made-up skin was absent. Thus, it was found that the desirable amount of applied titanium dioxide was 0.1-5 mg/cm².

### Relationship between the pigment refractive index and the hiding power

A test was conducted to investigate the relationship between the hiding power and the pigment refractive index. The results are shown in Table 10.

**(Table 10)**

| | 10-1 | 10-2 | 10-3 | 10-4 | 10-5 | 10-6 |
|---|---|---|---|---|---|---|
| Colorant composition | 5 | 5 | 5 | 5 | 5 | 5 |
| Titanium dioxide (refractive index 2.52-2.71) | 10 | - | - | - | - | - |
| Zinc oxide (refractive index 1.9-2.0) | - | 10 | 20 | - | - | - |
| Barium sulfate (refractive index 1.6) | - | - | - | 20 | - | - |
| Talc (refractive index 1.54-1.60) | - | - | - | - | 20 | - |
| Mica (refractive index 1.55-1.60) | - | - | - | - | - | 20 |
| Vaseline | 50 | 50 | 50 | 50 | 50 | 50 |
| Glyceryl tri(2-ethylhexanoatr) | 35 | 35 | 25 | 25 | 25 | 25 |
| Hiding power | ⊚ | Δ | ○ | × | × | × |
| Natural texture | ○ | ○ | ○ | ⊚ | ⊚ | ⊚ |

(Evaluation criteria for the hiding power)
⊚: concealed
○: somewhat concealed
Δ: barely concealed
×: not concealed
(Evaluation criteria of natural texture)
⊚: even and natural finish with a transparent feeling
○: somewhat natural finish
Δ: somewhat unnatural finish
×: unnatural finish

The values in the table are in wt%. The used colorant composition (before mixing with white pigment) was a mixture containing 0.13 wt% Lithol Rubine BCA (Toyo Ink Mfg. Co.) and 4.87 wt% tartrazine (Sun Chemical Corporation).

From the result, it was found necessary to use a high refractive index white pigment with a refractive index of 1.9 or higher in order to achieve satisfactory hiding power. In addition, it was found that titanium dioxide was especially desirable.

### Blending quantity of a white pigment of high refractive index and the blending ratio with a colorant composition

In order to investigate a desirable blending ratio of the colorant composition and the high refractive index white pigment (titanium dioxide) for the one-component type foundation of the present embodiment, samples were prepared according to the compositions (wt%) shown in the below-described table. The colorant composition before mixing with the white pigment is the same as that of Sample 5-3 in Table 5.

The test method is described below.

### (Preparation method of colorant composition)

Respective coloring materials (30 wt%) were dispersed in glyceryl tri(2-ethylhexanoate), kneaded three times with a three-roller mill to sufficiently disperse, and the dispersion was dispersed in the rest of glyceryl tri(2-ethylhexanoate). Then Vaseline was added to the dispersion, dissolved by heating, cooled in a water bath with stirring, and the obtained gel-like material was used as a sample.

**(Table 11A)**

| | 11-1 | 11-2 | 11-3 | 11-4 |
|---|---|---|---|---|
| Lithol Rubine BCA | 0.07 | 0.07 | 0.13 | 0.13 |
| Tartrazine | 2.43 | 2.43 | 4.87 | 4.87 |
| Titanium dioxide | 0.05 | 0.1 | 0.2 | 0.5 |
| Vaseline Glyceryl tri(2-ethylhexanoate) | 50 Balance | 50 Balance | 50 Balance | 50 Balance |
| Colorant/(tianium dioxide + colorant) (wt%) | 98% | 96.2% | 96.2% | 90.9% |
| Makeup color | ×red | Δred | ×red | Δred |
| Hiding power | × | Δ× | Δ× | Δ× |
| Natural texture | × | Δ | Δ | Δ |

**(Table 11B)**

| | 11-5 | 11-6 | 11-7 | 11-8 |
|---|---|---|---|---|
| Lithol Rubine BCA | 0.13 | 0.13 | 0.13 | 0.1.3 |
| Tartrazine | 4.87 | 4.87 | 4.87 | 4.87 |
| Titanium dioxide | 0.5 | 1 | 2 | 5 |
| Vaseline Glyceryl tri(2-ethylhexanoate) | 50 Balance | 50 Balance | 50 Balance | 50 Balance |
| Colorant/(titanium dioxide + colorant) (wt%) | 90.9% | 83.3% | 71.4% | 50.0% |
| Makeup color | ○red | ⊚thin | ⊚thin | ⊚ |
| Hiding power | Δ× | Δ | ○ | ⊚ |
| Natural texture | Δ | ○ | ⊚ | ⊚ |

**(Table 11C)**

| | 11-9 | 11-10 | 11-11 |
|---|---|---|---|
| Lithol Rubine BCA | 0.13 | 0.13 | 0.13 |
| Tartrazine | 4.87 | 4.87 | 4.87 |
| Titanium dioxide | 12.5 | 15 | 20 |
| Vaseline Glyceryl tri(2-ethylhexanoate) | 50 Balance | 50 Balance | 50 Balance |
| Coloran/(titamum dioxide + colorant) (wt%) | 28.6% | 25.0% | 20.0% |
| Makeup color | ⊚ | ⊚white | ⊚red |
| Hiding power | ⊚ | ⊚ | ⊚ |
| Natural texture | ⊚ | ⊚ | ⊚ |

**(Table 11D)**

| | 11-12 | 11-13 | 11-14 |
|---|---|---|---|
| Lithol Rubine BCA | 0.13 | 0.13 | 0.13 |
| Tartrazine | 4.87 | 4.87 | 4.87 |
| Titanium dioxide | 25 | 30 | 40 |
| Vaseline Glyceryl tri(2-ethylhexanoate) | 50 Balance | 50 Balance | 50 Balance |
| Colorant/(titanium dioxide + colorant) (wt%) | 16.7% | 14.3% | 11.1% |
| Makeup color | ○white | ○white | ×white |
| Hiding power | ⊚ | ⊚ | ⊚ |
| Natural texture | ⊚ | ○ | × |

The evaluation criteria for the makeup color, hiding power, and natural texture are as follows.

(Makeup color)
× red: unnaturally red
× white: unnaturally white
Δred: somewhat unnaturally red (for a person with considerably deep skin color)
Δwhite: somewhat unnaturally white (for a person with considerably light skin color)
Ored: reddish for the normal skin, but good for a person with deep skin color
Owhite: whitish for the normal skin, but good for a person with white skin color
⊚ : very natural and easy to apply (for a person with the normal skin)
⊚ thin: very natural (thinly apply for normal skin color)
⊚ red: very natural (for a person with somewhat deep skin color)
⊚ white: very natural (for a person with somewhat white skin color)

(Hiding power)
⊚: high hiding power
○: somewhat high hiding power
Δ: somewhat low hiding power
Δ×: low hiding power
×: no hiding power

(Natural texture)
⊚: even and natural finish with a transparent feeling
○: somewhat natural finish
Δ: somewhat unnatural finish
×: unnatural finish

As shown in Tables 11 A to 11 D, tests were conducted by varying the amount of titanium oxide in the base from 0.05 wt% to 40 wt%. As seen from the table, when the concentration was less than 1 wt% (Samples 11-1 to 11-5), the hiding power was not satisfactory, and the concealing effect for the troubled skin could not be expected. On the other hand, when the concentration was 25 wt% or higher (Samples 11-12 to 11-14), the hiding power was satisfactory. However, the applied color was whitish and unnatural for a person with a somewhat deep skin color. Therefore, the concentration should preferably be 1-25%, and more preferably 2-25%.

The blending ratio of a nonwhite coloring material and a high refractive index white pigment was investigated by varying the wt% (total nonwhite coloring material!(titanium dioxide + total nonwhite coloring material)) from 11.1% to 98%. As seen from the table, if the amount of the coloring material was larger than 90% (Samples 11-1 to 11-5), the applied color became reddish. Therefore, it was necessary to thinly apply a coating; as a result, the hiding power became low. On the other hand, if the amount of the coloring material was smaller than 20% (Samples 11-12 to 11-14), the applied color became white. Therefore, the color was unnatural except for a person with white skin color. Accordingly, the coloring material should be preferably 20-90%, and more preferably 20-70%.

Hereinafter, the present invention will be explained by means of some Examples, but the scope of this invention should not be limited to those Examples. In addition, all of the values in the formulation of those Examples are in wt%.

### Example 1 Two-component foundation

Formulation examples of two-component foundation, in which a combination of a colorant composition and a white pigment composition is used, are shown in Example 1. The white pigment compositions and the colorant compositions for skin cosmetics, in the below-described Example 1, can be used in any combination.

Oil-based white pigment composition
(Formulation)
Dimethylpolysiloxane 5
Isostearic acid 0.5
Diisostearyl malate 3
Glyceryl tri(2-ethylhexanoate) 1
Sorbitan sesquiisostearate 1
Alkyl-modified silicone resin coated titanium oxide 10
Methylhydrogenpolysiloxane coated talc balance
Methylhydrogenpolysiloxane coated sericite 20
N-lauroyl-L-lysine 0.1
Metal soap treated talc 8
Spherical silica 5
Vitamin E acetate 0.1
Ethyl paraben appropriate amount
Methyl bis(trimethylsiloxy)silyl isopentyl trimethoxycinnamate 1
2-Ethylhexyl para-methoxycinnamate 3
Spherical alkyl polyacrylate powder 6
(Preparation method)

The oil phase portion that was solubilized by heating at 80 degrees C was added to the powder portion in the formulation, and the mixture was blended with a Henschel mixer and pulverized with a pulverizer. The obtained powder was filled into a medium resin plate and press-formed to obtain an oil-based white pigment composition.

W/O cream-type white pigment composition
(Formulation)
<Oil phase>
Dimethylpolysiloxane 4
Decamethylpentasiloxane 25
Polyoxyethylene/methyl polysiloxane copolymer 4
Distearyldimethylammonium chloride 0.1
< Powder portion >
Alkyl-modified silicone resin coated titanium oxide 10
Alkyl-modified silicone resin coated sericite 10
N-lauroyl-L-lysine 0.1
P-hydroxybenzoic ester appropriate amount
<Water phase>
Propylene glycol 5
Water balance
(Preparation method)

The powder portion in the formulation was added to the oil phase portion that was solubilized by heating at 80 degrees C, and the mixture was pulverized with a homo mixer for 5 minutes. Then the water phase portion was added to the above-obtained mixture of the powder and oil, and the mixture was emulsified with the homo mixer for 5 minutes to obtain a W/O cream-type white pigment composition.

Powder-type colorant composition
(Formulation)
Dimethylpolysiloxane 5
Isostearic acid 0.5
Diisostearyl malate 3
Glyceryl tri(2-ethylhexanoate) 1
Sorbitan sesquiisostearate 1
Sunset Yellow FCF (Sun Chemical Corporation) 6.32
Tartrazine aluminum lake (Sun Chemical Corporation) 3.68
Methylhydrogenpolysiloxane coated talc balance
Methylhydrogenpolysiloxane coated sericite 20
N-lauroyl-L-lysine 0.1
Metal soap treated talc 8
Spherical silica 5
Vitamin E acetate 0.1
Ethyl paraben appropriate amount
Methyl bis(trimethylsiloxy)silyl isopentyl trimethoxycinnamate 1
2-Ethylhexyl para-methoxycinnamate 3
Spherical alkyl polyacrylate powder 6
(Preparation method)

The oil phase portion that was solubilized by heating at 80 degrees C was added to the powder portion in the formulation, and the mixture was blended with a Henschel mixer and pulverized with a pulverizer. The obtained mixture was filled into a medium resin plate and press-formed to obtain a powder-type colorant composition. The minimum spectral reflectance for this colorant composition was 80% in the range of 630-700 nm, and the Y value was 45.

W/O type emulsified solid colorant composition
(Formulation)
<Oil phase>
Microcrystalline wax 5
Dimethylpolysiloxane 10
Decamethylcyclopentasiloxane 30
Polyoxyethylene/methyl polysiloxane copolymer 2
Palmitic acid 0.5
Sorbitan sesquiisostearate 1
Tocopherol acetate 0.1
Delta-tocopherol 0.1
<Powder portion>
Lithol Rubine BCA (Toyo Ink Mfg. Co.) 0.25
Tartrazine aluminum lake (Sun Chemical Corporation) 9.75
Alkyl-modified silicone resin coated silicic acid anhydride 2
Alkyl-modified silicone resin coated sericite 15
Cross-linked silicone powder (Trefil E-506) 3
N-lauroyl-L-lysine 0.1
P-hydroxybenzoic ester appropriate amount
<Water phase>
Dipropylene glycol 3
Purified Water balance
(Preparation method)

The powder portion in the formulation was added to the oil phase portion that was solubilized by heating at 80 degrees C, and the mixture was pulverized with a homo mixer for 5 minutes. Then the water phase portion was added to the above-obtained mixture of the powder and oil, and the mixture was emulsified with the homo mixer for 5 minutes. The obtained mixture was filled into a medium resin plate to obtain a W/O type emulsified solid colorant composition. The minimum spectral reflectance for this colorant composition was 80% in the range of 630-700 nm, and the Y value was 50.

W/O cream-type colorant composition
(Formulation)
<Oil phase>
Dimethylpolysiloxane 4
Decamethylcyclopentasiloxane 25
Polyoxyethylene/methyl polysiloxane copolymer 4
Distearyldimethylammonium chloride 0.1
<Powder portion>
Lithol Rubine BCA (Toyo Ink Mfg. Co.) 0.17
Tartrazine aluminum lake (Kishi Kasei Co.) 9.83
Alkyl-modified silicone resin coated sericite 10
N-lauroyl-L-lysine 0.1
P-hydroxybenzoic ester appropriate amount
<Water phase>
Propylene glycol 5
Water balance
(Preparation method)

The powder portion in the formulation was added to the oil phase portion that was solubilized by heating at 80 degrees C, and the mixture was pulverized with a homo mixer for 5 minutes. Then the water phase portion was added to the above-obtained mixture of the powder and oil, and the mixture was emulsified with the homo mixer for 5 minutes to obtain a W/O cream-type colorant composition. The minimum spectral reflectance for this colorant composition was 79% in the range of 630-700 nm, and the Y value was 36.

Stick-type colorant composition
(Formulation)
<Oil phase>
Microcrystalline wax 12
Dimethylpolysiloxane 4
Decamethylcyclopentasiloxane 17
Glyceryl tri(2-ethylhexanoate) 26
Polyoxyethylene/methyl polysiloxane copolymer 4
<Powder portion>
Sunset Yellow FCF (Sun Chemical Corporation) 14
Hansa Yellow mica base (colorant content of 25%) (Kishi Kasei Co.) 24
(Preparation method)

The powder portion in the formulation was added to the oil phase portion that was solubilized by heating at 90 °C, and the mixture was pulverized with a homomixer for 5 minutes. Then the mixture was poured into a stick-shaped mold to obtain a stick-type colorant composition. The minimum spectral reflectance for this colorant composition was 80% in the range of 630-700 nm, and the Y value was 46.

Even-surfaced natural finish with an excellent transparent feeling could be achieved from any combination of a white pigment composition and a colorant composition listed in Example 1.

Example 2 One-component powdery foundation
(Formulation)
Dimethylpolysiloxane 5
Isostearic acid 0.5
Diisostearyl malate 3
Glyceryl tri(2-ethylhexanoate) 1
Sorbitan sesquiisostearate 1
Lithol Rubine BCA (Toyo Ink Mfg. Co.) 0.13
Tartrazine aluminum lake (Sun Chemical Corporation) 4.87
Spherical PMMA coated mica 6
Zinc oxide fine powder 0.5
Titanium oxide fine powder 2
Synthetic brown mica 2
Metal soap treated talc 8
Spherical silica 5
Vitamin E acetate 0.1
Ethyl paraben appropriate amount
Methyl bis(trimethylsiloxy)silyl isopentyl trimethoxycinnamate 1
2-Ethylhexyl para-methoxycinnamate 3
Spherical alkyl polyacrylate powder 6
Methylhydrogenpolysiloxane coated talc balance
Methylhydrogenpolysiloxane coated sericite 20
Methylhydrogenpolysiloxane coated titanium oxide 15
(Preparation method)

The oil phase portion that was solubilized by heating at 80 degrees C was added to the powder portion in the formulation, and the mixture was blended with a Henschel mixer and pulverized with a pulverizer. The obtained powder was filled into a medium resin plate and press-formed to obtain a powdery foundation.

Before mixing with a white pigment, the minimum spectral reflectance in the range of 630-700 nm was 80%, and the Y value was 50. Even-surfaced natural finish with an excellent transparent feeling could be achieved by the powdery foundation of Example 2.

Comparative Example 1 One-component powdery foundation
(Formulation)
Dimethylpolysiloxane 5
Isostearic acid 0.5
Diisostearyl malate 3
Glyceryl tri(2-ethylhexanoate) 1
Sorbitan sesquiisostearate 1
Spherical PMMA coated mica 6
Particulate zinc oxide 0.5
Particulate titanium oxide 2
Synthetic brown mica 2
Metal soap treated talc 8
Spherical silica 5
Vitamin E acetate 0.1
Delta-tocopherol 0.1
Ethyl paraben appropriate amount
Methyl bis(trimethylsiloxy)silyl isopentyl trimethoxycinnamate 1
2-Ethylhexyl para-methoxycimiamate 3
Spherical alkyl polyacrylate powder 6
Methylhydrogenpolysiloxane coated talc balance
Methylhydrogenpolysiloxane coated sericite 20
Methylhydrogenpolysiloxane coated titanium oxide 15
Alkyl-modified silicone resin coated yellow iron oxide 3
Alkyl-modified silicone resin coated colcothar 1
Alkyl-modified silicone resin coated black iron oxide appropriate amount
(Preparation method)

The oil phase portion that was solubilized by heating at 80 degrees C was added to the powder portion in the formulation, and the mixture was blended with a Henschel mixer and pulverized with a pulverizer. The obtained powder was filled into a medium resin plate and press-formed to obtain a powdery foundation. Before mixing with a white pigment, the minimum spectral reflectance in the range of 630-700 nm was 60%, and the Y value was 38.

When the powdery foundation of Comparative Example 1 was used, an uncomfortable feeling was caused by skin pores and small surface irregularities, and the finish was very powdery

Example 3 W/O type emulsified solid one-component type foundation
(Formulation)
<Oil phase>
Microcrystalline wax 5
Dimethylpolysiloxane 10
Decamethylcyclopentasiloxane 30
Polyoxyethylene/methyl polysiloxane copolymer 2
Palmitic acid 0.5
Sorbitan sesquiisostearate 1
Tocopherol acetate 0.1
Delta-tocopherol 0.1
<Powder portion>
Lithol Rubine BCA (Toyo Ink Mfg. Co.) 0.17
Tartrazine aluminum lake (Sun Chemical Corporation) 9.83
Alkyl-modified silicone resin coated silicic acid anhydride 2
Alkyl-modified silicone resin coated titanium oxide 15
Alkyl-modified silicone resin coated sericite 10
Cross-linked silicone powder (Trefil E-506) 3
N-lauroyl-L-lysine 0.1
P-hydroxybenzoic ester appropriate amount
<Water phase>
Dipropylene glycol 3
Purified Water balance
(Preparation method)

The powder portion in the formulation was added to the oil phase portion that was solubilized by heating at 80 degrees C, and the mixture was pulverized with a homo mixer for 5 minutes. Then the water phase portion was added to the above-obtained mixture of the powder and oil, and the mixture was emulsified with the homo mixer for 5 minutes. The obtained mixture was filled into a medium resin plate to obtain a W/O type emulsified solid foundation.

Here, the blending percentages of sunset yellow and tartrazine, which are nonwhite coloring materials, were the same as those of Sample 6-5 in Table 6. Before mixing with a white pigment, the minimum spectral reflectance in the range of 630-700 nm was 79%, and the Y value was 36. Even-surfaced natural finish with an excellent transparent feeling could be achieved by the W/O type emulsified solid foundation of Example 3.

Comparative Example 2 W/O type emulsified solid one-component type foundation
(Formulation)
<Oil phase>
Microcrystalline wax 5
Dimethylpolysiloxane 10
Decamethylcyclopentasiloxane 30
Polyoxyethylene/methyl polysiloxane copolymer 2
Palmitic acid 0.5
Sorbitan sesquiisostearate 1
Tocopherol acetate 0.1
Delta-tocopherol 0.1
<Powder portion>
Alkyl-modified silicone resin coated yellow iron oxide 3
Alkyl-modified silicone resin coated colcothar 1
Alkyl-modified silicone resin coated black iron oxide appropriate amount
Alkyl-modified silicone resin coated silicic acid anhydride 2
Alkyl-modified silicone resin coated titanium oxide 15
Alkyl-modified silicone resin coated sericite 10
Titanium oxide/colcothar coated mica 3
Cross-linked silicone powder (Trefil E-506) 3
N-lauroyl-L-lysine 0.1
P-hydroxybenzoic ester appropriate amount
<Water phase>
Dipropylene glycol 3
Purified Water balance
(Preparation method)

The powder portion in the formulation was added to the oil phase portion that was solubilized by heating at 80 degrees C, and the mixture was pulverized with a homo mixer for 5 minutes. Then the water phase portion was added to the above-obtained mixture of the powder and oil, and the mixture was emulsified with the homo mixer for 5 minutes. The obtained mixture was filled into a medium resin plate to obtain a W/O type emulsified solid foundation. Before mixing with a white pigment, the minimum spectral reflectance in the range of 630-700 nm was 59%, and the Y value was 37.

When the powdery foundation of Comparative Example 2 was used, an uncomfortable feeling was caused by skin pores and small surface irregularities, and the finish was very powdery.

## Claims

1. A colorant composition for skin cosmetics, wherein the colorant composition, which contains nonwhite coloring materials, does not contain a high refractive index white pigment, and when the spectral reflectance of the colorant composition is measured under the below-described measurement conditions, the minimum spectral reflectance on the white ground in the wavelength range of 630-700 nm is 75% or higher, and the Y value on the white ground is in the range of 25-65.
(Measurement conditions)
A colorant composition is dispersed in nitrocellulose lacquer, the concentration of the colorant composition is adjusted so that the spectral reflectance on the white ground of a black and white hiding test paper at the wavelength of 400 nm is 10±2%, a black and white hiding test paper is coated with an applicator having a 0.101 mm clearance, and the spectral reflectance is measured with a spectrocolorimeter.

2. The colorant composition for skin cosmetics according to claim 1, wherein nonwhite coloring materials contained in the colorant composition are plural coloring materials selected from the group consisting of Lithol Rubine BCA, Sunset Yellow FCF, tartrazine, and Hansa Yellow.

3. A multi-component type foundation, wherein a white pigment composition containing a high refractive index white pigment and a colorant composition according to either claim 1 or claim 2 are components.

4. The multi-component type foundation according to claim 3, wherein the white pigment composition and the colorant composition are mixed prior to the application to the skin.

5. A method of makeup application, wherein the method comprises the application step of a white pigment composition containing a high refractive index white pigment on the skin and the application step of a colorant composition according to either claim 1 or claim 2 on the skin.

6. The method of makeup application according to claim 5, wherein the amount of an applied high refractive index white pigment on the skin is 0.1-5 mg/cm² in total.

7. A one-component type foundation containing a nonwhite coloring material and a high refractive index white pigment, wherein when the spectral reflectance of the foundation-contained portion that is free from the high refractive index white pigment is measured under the below-described measurement conditions, the minimum spectral reflectance on the white ground in the wavelength range of 630-700 nm is 75% or higher, and the Y value on the white ground is in the range of 25-65;
wherein the measurement conditions are that the foundation-contained portion that is free from the high refractive index white pigment is dispersed in nitrocellulose lacquer, the concentration of the colorant composition is adjusted so that the spectral reflectance on the white ground of a black and white hiding test paper at the wavelength of 400 nm is 10±2%, a black and white hiding test paper is coated with an applicator having a 0.101 mm clearance, and the spectral reflectance is measured with a spectrocolorimeter.

8. The one-component type foundation according to claim 7, wherein the ratio of the total nonwhite coloring material and the high refractive index white pigment that are blended in the foundation (total nonwhite coloring material/(high refractive index white pigment + total nonwhite coloring material)) is 20-90 wt%.

9. The one-component type foundation according to either claim 7 or claim 8, wherein the amount of blended high refractive index white pigment is 1-25 wt%.
